Europäisches Patentamt

European Patent Office

Office européen des brevets

(11) Publication number: **0 175 585 B1**

# EUROPEAN PATENT SPECIFICATION

(45) Date of publication of patent specification: **25.09.91**  (51) Int. Cl.⁵: **G01N 33/53**, G01N 21/64

(21) Application number: **85306711.4**

(22) Date of filing: **20.09.85**

(54) **Dielectric waveguide sensors and their use in immunoassays.**

(30) Priority: **21.09.84 US 652714**
**06.09.85 US 773074**

(43) Date of publication of application:
**26.03.86 Bulletin 86/13**

(45) Publication of the grant of the patent:
**25.09.91 Bulletin 91/39**

(84) Designated Contracting States:
**AT BE DE FR GB IT NL**

(56) References cited:
**CH-A- 629 596**      **DE-A- 3 215 484**
**US-A- 4 341 957**    **US-A- 4 399 099**
**US-A- 4 402 819**    **US-A- 4 447 546**

**CHLINICAL CHEMISTRY, 29/9, 1983, MICHAEL
J. SEPANIAK "Optical Fiber Fluoroprobes in
Clinical Analysis" pages 1678-1682**

(73) Proprietor: **Ciba Corning Diagnostics Corp.
63 North Street
Medfield Massachusetts 02052(US)**

(72) Inventor: **Keck, Donald Bruce
Chequers Circle
Big Flats New York(US)**
Inventor: **Love, Walter Francis
713 Algonquin Drive
Horseheads New York(US)**

(74) Representative: **Kyle, Diana et al
ELKINGTON AND FIFE Beacon House 113
Kingsway
London WC2B 6PP(GB)**

## Description

The present invention relates to novel dielectric waveguide (i.e., fiber optic) sensors for use in spectrophotometric assays of analytes in fluids. More particularly, the invention relates to the use of these sensors in immunoassays.

Optical waveguides have been-used in various analytical tests. For example, in an article entitled "Optical Fiber Fluoroprobes in Clinical Analysis", Clin. Chem, 29/9, pp 1678-1682 (1983), Michael J. Sepaniak et al. describe the use of quartz optical fluoroprobes. By incorporating a single fiber within a hypodermic needle, the authors have been able to obtain in vivo measurement of the fluorescence of various therapeutic drug analytes in interstitial body fluids. Sepaniak et al state that their probe must use a laser radiation source as a fluorescence exciter.

One of the fluoroprobe designs uses a capillary action design for sampling. A length of optical fiber is stripped of its protective coating and slid inside a standard glass capillary tube, touching the walls of the capillary tube at random but not extending the whole length of the tube. This assembly is placed within a hypodermic needle.

Immunoassays using optical waveguides have been disclosed in European Patent Applications 82201107.8 and 81310385.5 to Battelle Memorial Institute. The earlier 1931 application discloses a competitive immunoassay using fiber optics. More particularly, a glass single or multimode optical fiber having a core with an index of refraction ($N_1$) and a cladding with an index of refraction ($N_2$), where $N_1 > N_2$, is coated with an antibody ($A_b$) film to form a sensor fiber.

The immunoassay is done in three steps. First the sensor fiber is immersed into a fluid containing an antigen ($A_g$) analyte specific to $A_b$ plus a known amount of flourescent-labelled $A_g$. A fluorescent coating forms in proportion to the $A_g$ concentration. Then, an excitation radiation is propagated down the sensor fiber core at one end. The immunoassay relies upon "evanescent wave" phenomena, i.e., the electromagnetic field components which extend a short distance into the cladding, to interact with and excite the external $A_b$/tagged $A_g$ complex. Finally, fluorescence from the excited tagged complex is "reinjected" back into the propagated down the core where it is detected at the opposite end of the fiber. The fluorescence may be reflected and emerge from the output end where it can be separated and detected.

In a continuation-in-part application filed in 1982, Battelle describes how to control the penetration of the exciting evanescent wave into the analyte-containing fluid. Here, the index of refraction of the core $N_1$ is greater than that ($N_2$) of the fluid such that the ratio $N_1/N_2$ permits the evanescent wave to penetrate only to the thickness of the $A_b/A_g$ complex. Thinner layers of such a complex are said to require an index of refraction which would eliminate a glass cladding. The second Battelle application includes more types of immunoassay examples using fiber optics, specifically, "sandwich," "limited reagent," "direct," and "sequential saturation" immunoassays.

An immunoassay apparatus developed by T. Hirschfeld is disclosed in U.S. Patent No. 4,447,546 issued May 8, 1984, which employs total internal reflection at an interface between a solid phase and a fluid phase of lower index of refraction to produce an evanescent wave in the fluid phase. Fluorescence excited by the wave is observed at angles greater than the critical angle, by total reflection within the solid medium. The solid phase is arranged and illuminated to provide multiple total internal reflections at the interface. Typically, the solid phase is in the form of an optical fiber to which is immobilized a component of a complex formed in an immunochemical reaction. A fluorophore is attached to another component of the complex. The fluorescent labeled component may be either the complement to or the analog of the immobilized component, depending upon whether competive or sandwich assays are to be performed. In the case of competitive assays, the labelled component is typically preloaded to the immobilized component in a controlled concentration.

The fiber and the attached constituent of the assay are immersed in a fluid phase sample and the exciting illumination is injected into an input end of the fiber. The evanescent wave is used to excite fluorescence in the fluid phase, and that fluorescence which tunnels back into the solid phase (propagating in direction greater than the critical angle) is detected at the input end of the fiber.

The observed volume of sample is restricted not only by the rapid decay of the evanescent wave as a function of distance from the interface, but by an equally fast decrease with distance of the efficiency of tunneling, the more distant fluorophores not only being less intensely excited and thus fluorescing less, but their radiation is less efficiently coupled into the fiber. Consequently the effective depth of the sensed layer is much reduced compared to the zone observed by total reflection fluorescence alone, the coupling efficiency effectively scaling down the zone.

Multiple total internal reflections in the solid phase allow the illuminating beam to excite repeatedly an evanescent wave, thereby more efficiently coupling the small excitation source to the sample volume. This also increases the amount of sample sensed. The latter is also enhanced by diffusive circulation of the sample past the fiber surface and to which the material being assayed adheres by reaction as it passes. Diffusion makes the actually sampled layer thickness much larger than the thin surface layer.

All the radiation that tunnels back into the fibers is within the total reflection angle and is thus trapped within the fiber. The power available from the fluorescence increases with the length of fiber within the fluorescing material. However, the optical throughput of the system (determined by the aperture and the numerical aperture of the fiber) remains constant. The total fluorescent signal coming from the entire surface of the fiber, multiplied by the increase in sample volume due to diffusion, thus becomes available in a very bright spot (that is the cross-section of the fiber in diameter) exiting the fiber at its input end through a restricted angle determined by the critical angle of reflection within the fiber. Such signal is easily collected at high efficiency and throughput when matched to a small detector.

U.S. Patent No. 4,399,099 describes an optical fiber device for the quantitative analysis of an analyte in a fluid which comprises an energy transmissive core and one or more sheaths surrounding the core. The core is permeable or impermeable to different components of an aqueous fluid solution.

The object of the present invention is to provide an improved dielectric waveguide and, in particular a dielectric waveguide suitable for use in spectrophotometric assays of analytes in fluids, more particularly in immunoassays. A further object is to provide a dielectric waveguide which includes a light transmissive element having a cladding which has an index of refraction which is a less than the index of refraction of the light transmissive element whereby the cladding functions to contain substantially all of the exciting radiation within the light transmissive element.

A first aspect of the invention provides a dielectric waveguide for use in spectro-photometric assay of an analyte in a fluid comprising:

a) a hollow core having a longitudinal axial bore and an index of refraction $N_1$;

b) a cladding about the core having an index of refraction $N_2$ which is less than $N_1$; and

c) a reactant coating on the inner surface of the core which interacts with the analyte to affect electromagnetic radiation transmitted down the waveguide.

For descriptive purposes, the waveguide comprises a core transmissive to electromagnetic radiation, preferably visible light, having an index of refraction ($N_1$) and an opening in the core. The core thickness is sufficient to propagate the exciting radiation substantially down the core. A cladding with an index of refraction $N_2$ (which is less than $N_1$) is about the outside of the core. The cladding is thick enough to contain substantially all of the exciting radiation launched below the critical angle of the waveguides, but to permit penetration of the evanescent wave into a reactant coating. Finally, a reactant coating is placed about the core opening which, in the presence of electromagnetic radiation, interacts with the analyte to form a signal radiation.

The light propagation in this and the other waveguide structures to be discussed consists of modes with propagation constant, $\beta$, such that $E \approx e^{ie}$, where E is the lightwave electric field amplitude and z the distance along the waveguide. Oscillatory solutions for E, i.e., bound modes, are obtained for $N_2 k < \beta < N_1 k$ where $k = \frac{2\pi}{\lambda}$ and is the free space wavelength of the light. Leaky modes for which $N_3 k < \beta < N_2 k$ are also obtained but these generally decay with length z (where $N_3$ is the index of refraction of the fluid surrounding the waveguide). With a suitable combination of spot size and launch angle, the penetration of light into the analyte can be controlled.

For example, if $N_2 = N_3$ for simplicity, then the extension of the electric field into the analyte is given by:

$$E - K\nu(\gamma r) \text{ for } r > a$$

where 2a denotes the thickness of the core region, $\gamma$ is the mode number, $\gamma = (N_1^2 k^2 - \beta^2)^{1/2}$, and K is the modified Hankel function. This applies strictly to the case of a concentric circular fiber but may be used approximately here. The mathematical matching of this evanescent electric field to the core mode electric field gives the value of $\gamma$. For the lowest order mode, vis., $\nu = 0$,

$$E - \frac{e^{-\gamma r}}{r} \text{ for } r > a$$

Thus, the penetration distance of the light into the analyte depends on $\gamma$ which in turn depends on $\lambda$ the mode(s) selected by the launch (initial) conditions ($\nu$), the indices of refractions of the waveguide ($N_1$ and $N_2$) and analyte ($N_3$), and the wavelength of the light ($\lambda$).

The above hollow waveguide can be used in the following manner. The coated waveguide is placed in the analyte-containing fluid for a time sufficient for the analyte to interact with the reactant coating and to form an electromagnetically detectable complex or moeity. Then either while the fiber is still in the fluid or after it has been removed, electromagnetic radiation is propagated down the waveguide core so as to irradiate the interacting moeity, which then produces a signal radiation. The last step is to detect the resulting signal radiation by monitoring the core of the waveguide. Typically, the waveguide is a fiber having two ends, either one of which can be monitored.

According to a second aspect of the invention there is provided a multi-element dielectric waveguide for use in a spectrophotometric assay of an analyte in a fluid comprising:

a) a hollow, first, support, fiber having a longitudinal axial bore and an index of refraction $N_A$;

b) a second, core, fiber axially positioned within the hollow, first, core, fiber and having an index of refraction $N_B$;

c) a cladding provided on the second, core, fibre, said cladding having an index of refraction $N_C$ which is greater than $N_B$;

d) a means for maintaining the axial position of the second, core, fiber within the hollow, first, core, fiber; and

e) a reactant coating on the cladding of its second, core, fibre which interacts with the analyte to affect electromagenetic radiation transmitted down the waveguide.

The relationships of $N_A/N_B$ depends upon how one intends on using the waveguide in an assay. $N_B$ can be either greater than, equal to, or less than $N_A$. The selection of material and waveguide design parameters such as thickness, follow principles either known to the art or described above.

There are three general methods of using the multi-element dielectric waveguide. In the first, the exciting radiation is propagated down the core fiber. The evanescent wave from this propagation interacts with either the analyte itself or the combination of analyte and reactant coating on either of the fibers to produce a signal radiation. Either the core or the support fiber can be monitored to detect the signal radiation, however, the detecting waveguide should have an index of refraction equal to or greater than the excitating waveguide.

Another method uses the hollow support waveguide to propagate the excitation radiation. Again, either waveguide can be used for detection, but the detecting waveguide should have an index of refraction equal to or greater than the exciting waveguide.

The third method does not use the waveguide as an exciter. Rather, the analyte-containing fluid is used as the propagating medium for the excitation radiation. Either waveguide is used for detection. Of course, the fluid must be able to propagate the excitation radiation.

A third aspect of the invention is defined in claim 19. Such configurations are known to the art as Bragg waveguides. The selection materials and design parameters such as thickness, follow principles either known to the art or described above.

For assay purposes, one coats the interior core surface of a Bragg waveguide with a reactant which, in the presence of electromagnetic radiation, interacts with the analyte to form a detectable signal radiation.

The coated Bragg waveguide can be used in an assay in a method similar to the first hollow waveguide; however, the excitation and signal radiation are both launched and carried down the opening of the core fiber rather than the fiber itself.

Of course, an apparatus useful for practicing the above method would include the following elements: an electromagnetic radiation source; a means for guiding the radiation from the source to the interior of the waveguide, where it is propagated, a signal radiation detection means; and a means for guiding the signal radiation from the waveguide, to the detection means. All of these means are conventional and well known to the skilled artisan.

FIGURE 1 is a cross-sectional view of a hollow waveguide.

FIGURE 2 is a cross-sectional view of a multi-element waveguide.

FIGURE 3 is a cross-sectional view of a Bragg waveguide.

FIGURE 4 is a diagrammatic view of an apparatus for use with the above waveguides.

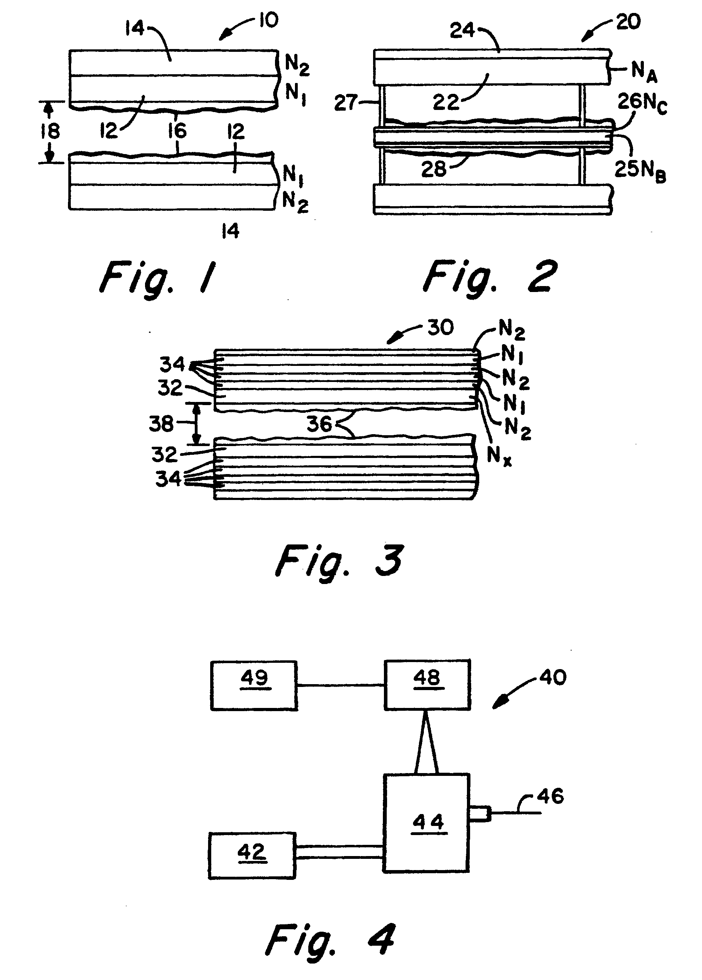

A preferred embodiment of a hollow waveguide is shown in Figure 1. The waveguide 10 comprises a hollow glass cylindrical core 12 having an index of refraction $N_1$, an internal core diameter of about 100 microns, and a thickness of about 250 microns. The core is covered on the outside by a glass cladding 14 having an index of refraction $N_2$, which is less than $N_1$, and a thickness of about 250 microns. Those skilled in the art of optical fibers know how to select suitable optically transmissive materials, such as glass or plastic, and how to make such a structure, therefore a detailed description of the various processes is superfluous. However, the following disclosures are given as exemplary references on both multi-mode and single mode waveguide construction: U.S. 3,695,915 to Maurer et al; U.S. 3,711,262 to Keck et al; U.S. 3,775,075 to Keck et al; and U.S. 3,823,995 to Carpenter.

The interior surface of the waveguide core is covered with an immobilized reactant coating 16. The chemical composition of this coating varies according to the type of analyte being detected and the type of signal radiation one is trying to generate. As for analytes suitable for detection with the present waveguides, the main requirement is for the reactant coating to be able to bind the analyte directly. For example, if the analyte is an immunological substance (i.e., antibody, antigen, or hapten), then the reactant coating comprises a complementary immunological substance which is secured to the core yet able to bind to the analyte. Thus, an antigen (Ag) analyte would require a complementary antibody ($A_b$) component to be immobilized to the core as the reactant coating.

Those of skill in the immunoassay art have applied the selective binding property of antibodies to create different types of immunoassays known as "sandwich", "direct", "limited reagent" and "saturation" assays. See U.S. 4,380,580. The skilled artisan would know how to design an immunoassay by selecting the proper immunological substances for a reactant coating that would be suitable for use on the present coated, hollow waveguides.

Signal radiation selection can affect the selection of the reactant coating as well. For example, if chemiluminescent production of a particular signal is desired in an immunoassay, then the reactant coating can comprise an immobilized chemiluminescent precursor or reactant which, in the presence of the analyte, results in the production of this signal. Alternatively, the precursor can be used according to the methods disclosed in U.S. 4,380,580, where the chemiluminescent precureor is attached to either an antibody or an antigen which would react with the coating. These configurations are opposed to immunoassays where, if fluorescence is the signal to be monitored, then the art knows how to apply fluorescent "tags" either to the analyte or to a competitive analyte (or analogue thereof) without affecting the makeup of the reactant coating.

If desired, a mirror coating (not shown) can be applied to the outside of the cladding. The effect would be to reflect the isotropic signal radiation so as to permit more Of the signal to be propagated back down the waveguide.

The multi-element waveguide is illustrated in Figure 2. Preferably, the waveguide comprises two spaced fibers. A hollow, cylindrical support fiber 22 having an index of refraction $N_A$, an interior diameter of 1000 $\mu$m (microns), and a thickness of 250 $\mu$m (microns) is coated with a reflective, mirror layer 24. Positioned within the interior of the support fiber is a core fiber 25 having an index of refraction $N_B$ and a thickness of 250 microns, which has a cladding 26 about the core having an index of refraction $N_C$ and a thickness of 50 microns. $N_B$ is greater than $N_A$ and less than $N_C$ if the core fiber is used for detection. Spacer means 27 comprising at least en annuler ring keeps the core fiber axially and concentrically positioned within the length of the hollow support fiber.

Finally, a reactant coating 28 covers the cladding surface of the core fiber. Again, as discussed above, this coating can have variable compositions.

In the arrangement of Figure 3, the third Bragg waveguide 30 has a glass hollow cylindrical core 32 with an interior diameter of 1000 microns and an index of refraction $N_X$, surrounded on the outside by a multicomponent cladding 34 and on the inside with a reactant coating 36 similar to the ones described above. The cladding comprises a series of alternating materials having indices of refraction $N_1$ and $N_2$, where $N_2 < N_1$ and only one of either $N_2$ or $N_1$ can equal $N_X$. The cladding thicknesses vary according to the indices of refraction, as mentioned herein.

In general, an apparatus for using these waveguides in spectrophotometric assays 40 has the five elements diagramatically presented in Figure 4. They are: an excitation radiation source 42; a means for guiding the excitation radiation 44 to the waveguide 46, either at the core, the cladding, or the hollow interior, where it is propagated; a signal radiation detection means 48; a means for guiding the signal radiation, also 44 from the waveguide to the signal detection means; and, preferably, a recordation and processing means 49 which can collect detection data in a more permanent form.

Most of these elements are standard features on spectrophotometers. For example, the exciter can be either a dye-tunable laser or a tungsten bulb. The guide means can comprise focusing lenses, monochromator gratings, mirrors, and wavelength selective beam splitters. Finally, the detector and recorder can be either a photomultiplier tube or a photo-diode and a microprocessor with storage and display abilities. The design of such an apparatus would be within the skill of on optics artisan.

An important aspect of any apparatus using the present waveguides is the waveguide alignment means. That is, part of the function of the guiding means is to ensure that the excitation radiation is propagated within the waveguide. Thus, according to known optical principles the waveguide must be properly aligned with this radiation, otherwise bound analyte will not be excited by an evanescent wave of the proper wavelength. More than one gripping arrangement can be used, from as simple as a matching cylindrical guide sheath to as complicated as movable opposing jaws with precision molded grips.

Having described the invention with particular reference to preferred embodiments, it will be obvious to those skilled in the art to which the invention pertain, that, after understanding the invention, various changes and modifications may be made without departing from the scope of the invention as defined by the appended claims.

## Claims

1. A dielectric waveguide for use in a spectrophotometric assay of an analyte in a fluid comprising:

a) a hollow core having a longitudinal axial bore and an index of refraction $N_1$;

b) a cladding about the core having an index of refraction $N_2$ which is less than $N_1$; and

c) a reactant coating on the inner surface of the core which, interacts with the analyte to affect electromagnetic radiation transmitted down the waveguide.

2. A dielectric waveguide according to claim 1, wherein the core is a hollow throughout its length.

3. A dielectric waveguide according to claim 1 or 2, wherein the waveguide has a fiber shape with two ends, one of which has a mirror coating.

4. A dielectric waveguide according to claim 1, wherein the waveguide has a fiber shape and a mirror coating on the cladding.

5. A dielectric waveguide according to any of claims 1 to 4, wherein the reactant coating is an immobilized antibody.

6. A dielectric waveguide according to any of claims 1 to 4, wherein the reactant coating is an immobilized antigen.

7. A dielectric waveguide according to any of claims 1 to 4, wherein the reactant coating is an enzyme.

8. A method of spectrophotometrically assaying an analyte in a fluid comprising:

a) contacting the waveguide of claim 1 with the fluid for a time sufficient for the analyte and immobilised reactant to be able to interact;

b) propagating radiation down the core so as to irradiate the interacting analyte and reactant; and

c) detecting the signal radiation resulting from the irradiation of the analyte interaction by monitoring the waveguide.

9. An improved apparatus for spectrophotometrically assaying an analyte in a fluid having an electromagnetic radiation source, and a means for detecting electromagnetic radiation, the improvement comprising:

a) the waveguide of claim 1;

b) a means for guiding electromagnetic radiation from the source, to the waveguide such that it is propagated down the waveguide; and

c) a means for guiding the electromagnetic signal radiation from the waveguide to the detection means.

10. A multi-element dielectric waveguide for use in a spectrophotometric assay of an analyte in a fluid comprising:

a) a hollow, first, support, fiber (22) having a longitudinal axial bore and an index of refraction $N_A$;

b) a second, core, fiber (25) axially positioned within the hollow first support fiber and having an index of refraction $N_B$;

c) a cladding (26) provided on the second, core, fiber, said cladding having an index of refraction $N_C$ which is greater than $N_B$.

d) a means for maintaining the axial position of the second, core, fiber within the hollow, first, support, fiber; and

e) a reactant coating (28) on the cladding of said second, core, fiber which interacts with the analyte to affect electromagnetic radiation transmitted down the waveguide.

11. A dielectric waveguide according to claim 10, wherein $N_B$ is greater than $N_A$.

12. A dielectric waveguide according to claim 10, wherein $N_B$ is equal to $N_A$.

13. A dielectric waveguide according to claim 10, wherein $N_B$ is less than $N_A$.

14. A dielectric waveguide according to any of claims 10 to 13, wherein a mirror coating is placed about the inside of the hollow first support fiber.

15. A dielectric waveguide according to any of claims 10 to 13, wherein a mirror coating is placed about the outside of the hollow first support fiber.

16. A dielectric waveguide according to any of claims 10 to 15 which comprises a reactant coating on the hollow first support fiber which interacts with the analyte to affect electromagnetic radiation transmitted down the waveguide.

17. A method of spectrophotometrically assaying an analyte in a fluid comprising:

a) contacting the waveguide of claim 10 with the fluid for a time sufficient for the analyte and immobilised reactant to be able to interact;

b) propagating radiation drown the second core fiber so as to irradiate the combination of analyte and immobilised reactant; and

c) detecting the signal radiation from the irradiated analyte and reactant interaction by monitoring the waveguide.

18. A method of spectrophotometrically assaying an analyte in a fluid comprising;

a) contacting the waveguide of claim 10 with the fluid for a time sufficient for the analyte and immobilised reactant to be able to interact;

b) propagating radiation down the hollow first core fiber so as to irradiate the combination of analyte and immobilised reactant; and

c) detecting the signal radiation from the irradiated analyte and reactant interaction by monitoring the waveguide.

19. A dielectric waveguide for use in a spectrophotometric assay of an analyte in a fluid wherein a signal radiation is propagated in the fluid comprising:

a) a hollow core having a longitudinal axial bore and an index of refraction $N_X$;

b) a series of claddings about the core having alternating indices of refraction $N_2$ and $N_1$ where $N_2$ is less than $N_1$ and only one of either can equal $N_X$, and of such a number and configuration so as to enable electromagnetic radiation to propagate within the core and

c) a reactant coating on the inner surface of the core which interacts with the analyte to affect electromagnetic radiation transmitted down the waveguide.

20. A method of spectrophotometrically assaying an analyte in a fluid comprising:

a) contacting the waveguide of claim 19 with the fluid for a time sufficient for the analyte and immobilised reactant to interact;

b) propagating radiation down the waveguide so as to irradiate the combination of analyte and immobilised reactant; and

c) detecting the signal radiation resulting from the irradiation of the analyte and reactant interaction by monitoring the fluid.

**Revendications**

1. Guide d'ondes diélectrique destiné à être utilisé pour un essai spectrophotométrique d'un analyte dans un fluide, comprenant :

a) un coeur creux possédant un perçage axial longitudinal et un indice de réfraction $N_1$;

b) une gaine disposée autour du coeur et possédant un indice de réfraction $N_2$ inférieur à $N_1$; et

c) un revêtement réactif situé sur la surface intérieure du coeur et qui réagit avec l'analyte pour modifier un rayonnement électromagnétique transmis dans le guide d'ondes.

2. Guide d'ondes diélectrique selon la revendication 1, dans lequel le coeur est creux sur toute sa longueur.

3. Guide d'ondes diélectrique selon la revendication 1 ou 2, dans lequel le guide d'ondes possède la forme d'une fibre ayant deux extrémités dont l'une possède un revêtement réfléchissant.

4. Guide d'ondes diélectrique selon la revendication 1, dans lequel le guide d'ondes possède la forme d'une fibre et un revêtement réfléchissant est placé sur la gaine.

5. Guide d'ondes diélectrique selon l'une quelconque des revendications 1 à 4, dans lequel le revêtement réactif est un anticorps immobilisé.

6. Guide d'ondes diélectrique selon l'une quelconque des revendications 1 à 4, dans lequel le revêtement réactif est un antigène immobilisé.

7. Guide d'ondes diélectrique selon l'une quelconque des revendications 1 à 4, dans lequel le revêtement réactif est un enzyme.

8. Procédé d'essai spectrophotométrique d'un analyte dans un fluide, consistant à :

a) mettre en contact le guide d'ondes de la revendication 1 avec le fluide pendant un intervalle de temps suffisant pour que l'analyte et le réactif immobilisé puissent réagir entre eux;

b) amener un rayonnement à se propager dans le coeur afin d'irradier l'analyte et le réactif, qui réagissent entre eux; et

c) détecter le rayonnement de signal produit par l'irradiation de l'interaction avec l'analyte par contrôle du guide d'ondes.

9. Dispositif perfectionné pour l'essai spectrophotométrique d'un analyte dans un fluide, comportant une source de rayonnement électromagnétique, et des moyens pour détecter le rayonnement électromagnétique, le perfectionnement comprenant :

    a) le guide d'ondes de la revendication 1;

    b) des moyens pour guider un rayonnement électromagnétique depuis la source en direction du guide d'ondes de manière qu'il se propage le long de ce dernier; et

    c) des moyens pour guider le signal de rayonnement électromagnétique depuis le guide d'ondes jusqu'aux moyens de détection.

10. Guide d'ondes diélectrique à éléments multiples destiné à être utilisé pour un essai spectrophotométrique d'un analyte dans un fluide, comprenant :

    a) une première fibre de support creuse (22) possédant un perçage axial longitudinal et un indice de réfraction $N_A$ ;

    b) une seconde fibre formant coeur (25) disposée axialement à l'intérieur de la première fibre de support creuse et possédant un indice de réfraction $N_B$;

    c) une gaine (26) disposée sur la seconde fibre formant coeur, ladite gaine possédant un indice de réfraction $N_C$ supérieur à $N_B$ ;

    d) des moyens pour maintenir la position axiale de la seconde fibre formant coeur dans la première fibre creuse de support; et

    e) un revêtement réactif (28) disposé sur la gaine de ladite seconde fibre formant coeur et qui réagit avec l'analyte pour modifier le rayonnement électromagnétique transmis dans le guide d'ondes.

11. Guide d'ondes diélectrique selon la revendication 10, dans lequel $N_B$ est supérieur à $N_A$.

12. Guide d'ondes diélectrique selon la revendication 10, dans lequel $N_B$ est égal à $N_A$.

13. Guide d'ondes diélectrique selon la revendication 10, dans lequel $N_B$ est inférieur à $N_A$.

14. Guide d'ondes diélectrique selon l'une quelconque des revendications 10 à 13, dans lequel un revêtement réfléchissant est disposé sur le pourtour intérieur de la première fibre de support creuse.

15. Guide d'ondes diélectrique selon l'une quelconque des revendications 10 à 13, dans lequel un revêtement réfléchissant est disposé sur le pourtour extérieur de la première fibre de support creuse.

16. Guide d'ondes diélectrique selon l'une quelconque des revendications 10 à 15, qui comporte un revêtement réactif situé sur la première fibre de support creuse et qui coopère avec l'analyte de manière à modifier le rayonnement électromagnétique transmis dans le guide d'ondes.

17. Procédé d'essai spectrophotométrique d'un analyte dans un fluide, consistant à :

    a) mettre en contact le guide d'ondes de la revendication 10 avec le fluide pendant un intervalle de temps suffisant pour que l'analyte et le réactif immobilisé puissent réagir entre eux;

    b) amener le rayonnement à se propager dans la seconde fibre formant le coeur de manière à irradier la combinaison de l'analyte et du réactif; et

    c) détecter le rayonnement de signal produit par l'irradiation de l'interaction entre l'analyte et le réactif par contrôle de guide d'ondes.

18. Procédé d'essai spectrophotométrique d'un analyte dans un fluide, consistant à :

    a) mettre en contact le guide d'ondes de la revendication 10 avec le fluide pendant un intervalle de temps suffisant pour que l'analyte et le réactif immobilisé puissent réagir entre eux;

    b) amener le rayonnement à se propager dans la première libre formant le coeur de manière à irradier la combinaison de l'analyte et le réactif; et

    c) détecter le rayonnement de signal produit par l'irradiation de l'interaction entre l'analyte et le réactif par contrôle du guide d'ondes.

19. Guide d'ondes diélectrique destiné à être utilisé pour un essai spectrophotométrique d'un analyte dans un fluide, et dans lequel un signal de rayonnement se propage dans le fluide, comprenant :

    a) un coeur creux possédant un perçage axial longitudinal et un indice de réfraction $N_X$;

    b) une série de gaines disposées autour du coeur et possédant des indices de réfraction alternés $N_2$ et $N_1$, $N_2$ étant inférieur à $N_1$ et seul l'un de ces indices pouvant être égal à $N_X$, ces indices de réfraction étant présent en un nombre et selon une disposition tels qu'ils permettent la propagation d'un rayonnement électromagnétique dans le coeur; et

    c) un revêtement réactif situé sur la surface intérieure du coeur et qui réagit avec l'analyte pour modifier le rayonnement électromagnétique transmis dans le guide d'ondes.

20. Procédé d'essai spectrophotométrique d'un analyte dans un fluide, consistant à :

a) mettre le guide d'ondes de la revendication 19 en contact avec le fluide pendant un intervalle de temps suffisant pour que l'analyte et le réactif immobilisé réagissent entre eux;

b) amener le rayonnement à se propager dans le guide d'ondes de manière à irradier la combinaison de l'analyte et du réactif immobilisé; et

c) détecter le rayonnement de signal résultant de l'irradiation de l'interaction entre l'analyte et le réactif, au moyen d'un contrôle du fluide.

## Patentansprüche

1. Dielektrischer Wellenleiter für die Verwendung in einem spektralphotometrischen Test eines Analyts in einem Fluid bestehend aus:

a) einem hohlen Kern, der eine axiale Längsbohrung und einen Brechungsindex $N_1$ aufweist;

b) einem um den Kern angeordneten Mantel, der einen Brechungsindex $N_2$ aufweist, der kleiner als $N_1$ ist; und

c) einer Reaktionsmittelschicht auf der inneren Oberfläche des Kerns, die mit dem Analyt in Wechselwirkung steht, um zu bewirken, daß elektromagnetische Strahlung durch den Wellenleiter übertragen wird.

2. Dielektrischer Wellenleiter gemäß Anspruch 1, bei dem der Kern über seine Länge hohl ist.

3. Dielektrischer Wellenleiter nach Anspruch 1 oder 2, bei dem der Wellenleiter die Form einer Faser mit zwei Enden aufweist, von denen ein Ende eine Spiegelbeschichtung aufweist.

4. Dielektrischer Wellenleiter nach Anspruch 1, wobei der Wellenleiter eine Faserform und eine Spiegelbeschichtung auf dem Mantel aufweist.

5. Dielektrischer Wellenleiter nach einem der Ansprüche 1 bis 4, bei dem die Reaktionsmittelschicht ein immobilisierter Antikörper ist.

6. Dielektrischer Wellenleiter nach einem der Ansprüche 1 bis 4, bei dem die Reaktionsmittelschicht ein immobilisiertes Antigen ist.

7. Dielektrischer Wellenleiter nach einem der Ansprüche 1 bis 4, bei dem die Reaktionsmittelschicht ein Enzym ist.

8. Verfahren zum spektralphotometrischen Testen eines Analyts in einem Fluid, bei dem

a) der Wellenleiter nach Anspruch 1 für eine ausreichende Zeit mit dem Fluid kontaktiert wird, so daß das Analyt und das immobilisierte Reaktionsmittel aufeinander einwirken können;

b) sich Strahlung in bzw. entlang dem Kern ausbreitet, um das Analyt und Reaktionsmittel, die in Wechselwirkung stehen, zu bestrahlen; und

c) die Signalstrahlung, die aus der Bestrahlung der Analyt-Wechselwirkung resultiert, durch Beobachtung des Wellenleiters erfaßt wird.

9. Verbesserte Einrichtung zum spektralphotometrischen Testen eines Analyts in einem Fluid, die eine elektromagnetische Strahlungsquelle und eine Vorrichtung zur Erfassung elektromagnetischer Strahlung aufweist, wobei die Verbesserung beinhaltet:

a) den Wellenleiter nach Anspruch 1;

b) eine Vorrichtung zur Führung elektromagnetischer Strahlung von der Quelle zu dem Wellenleiter, so daß sie sich in dem Wellenleiter ausbreitet; und

c) eine Vorrichtung zur Führung der elektromagnetischen Signalstrahlung von dem Wellenleiter zu der erfassenden Vorrichtung.

10. Aus vielen Elementen bestehender dielektrischer Wellenleiter für die Verwendung in einem spektralphotometrischen Test eines Analyts in einem Fluid, der aufweist:

a) eine hohle ersten Stützfaser (22), die eine axiale Längsbohrung und einen Brechungsindex $N_A$ aufweist;

b) eine zweite Kernfaser (25), die axial innerhalb der hohlen ersten Stützfaser angeordnet ist und einen Brechungsindex $N_B$ aufweist;

c) einen Mantel (26), der auf der zweiten Kernfaser angeordnet ist, wobei der Mantel einen Brechungsindex $N_C$ aufweist, der größer als $N_B$ ist;

d) eine Vorrichtung zur Aufrechterhaltung der axialen Position der zweiten Kernfaser innerhalb der hohlen ersten Stützfaser; und

e) eine Reaktionsmittelschicht (28) auf dem Mantel der zweiten Kernfaser, die mit dem Analyt in Wechselwirkung steht, um zu bewirken, daß elektromagnetische Strahlung durch den Wellenleiter übertragen wird.

**11.** Dielektrischer Wellenleiter nach Anspruch 10, bei dem $N_B$ größer als $N_A$ ist.

**12.** Dielektrischer Wellenleiter nach Anspruch 10, bei dem $N_B$ gleich $N_A$ ist.

**13.** Dielektrischer Wellenleiter nach Anspruch 10, bei dem $N_B$ kleiner als $N_A$ ist.

**14.** Dielektrischer Wellenleiter nach einem der Ansprüche 10 bis 13, bei dem eine Spiegelschicht auf der Innenseite der hohlen ersten Stützfaser angeordnet ist.

**15.** Dielektrischer Wellenleiter nach einem der Ansprüche 10 bis 13, bei dem eine Spiegelschicht um die Außenseite der hohlen ersten Stützfaser angeordnet ist.

**16.** Dielektrischer Wellenleiter nach einem der Ansprüche 10 bis 15, der eine Reaktionsmittelschicht auf der hohlen ersten Stützfaser aufweist, die mit dem Analyt in Wechselwirkung steht, um zu bewirken, daß elektromagnetische Strahlung durch den Wellenleiter übertragen wird.

**17.** Verfahren zum spektralphotometrischen Testen eines Analyts in einem Fluid, bei dem
a) der Wellenleiter nach Anspruch 10 für eine ausreichende Zeit mit dem Fluid kontaktiert wird, so daß das Analyt und das immobilisierte Reaktionsmittel aufeinander einwirken können;
b) sich Strahlung in der zweiten Kernfaser ausbreitet, um die Kombination aus Analyt und immobilisiertem Reaktionsmittel zu bestrahlen; und
c) die Signalstrahlung, die aus der bestrahlten Wechselwirkung von Analyt und Reaktionsmittel resultiert, durch Beobachtung des Wellenleiters erfaßt wird.

**18.** Verfahren zum spektralphotometrischen Testen eines Analyts in einem Fluid, bei dem
a) der Wellenleiter nach Anspruch 10 für eine ausreichende Zeit mit dem Fluid kontaktiert wird, so daß das Analyt und das immobilisierte Reaktionsmittel aufeinander einwirken können;
b) sich Strahlung in der hohlen ersten Kernfaser ausbreitet, um die Kombination aus Analyt und immobilisiertem Reaktionsmittel zu bestrahlen; und
c) die Signalstrahlung, die aus der bestrahlten Wechselwirkung von Analyt und Reaktionsmittel resultiert, durch Beobachtung des Wellenleiters erfaßt wird.

**19.** Dielektrischer Wellenleiter für die Verwendung in einem spektralphotometrischen Test eines Analyts in einem Fluid, bei dem sich eine Signalstrahlung in dem Fluid ausbreitet, bestehend aus:
a) einem hohlen Kern, der eine axiale Längsbohrung und einen Brechungsindex $N_X$ aufweist;
b) einer Serie von Mänteln auf dem Kern, die alternierende Brechungsindizes $N_2$ und $N_1$ aufweisen, wobei $N_2$ kleiner als $N_1$ ist und nur eine von beiden gleich $N_X$ sein kann, und solch eine Anzahl und einen Aufbau aufweist, daß elektromagnetische Strahlung befähigt wird, sich innerhalb des Kerns auszubreiten, und
c) einer Reaktionsmittelschicht auf der inneren Oberfläche des Kerns, die mit dem Analyt in Wechselwirkung steht, um zu bewirken, daß eletromagnetische Strahlung durch den Wellenleiter übertragen wird.

**20.** Verfahren zum spektralphotometrischen Testen eines Analyts in einem Fluid, bei dem
a) der Wellenleiter nach Anspruch 19 für eine ausreichende Zeit mit dem Fluid kontaktiert wird, so daß das Analyt und das immobilisierte Reaktionsmittel in Wechselwirkung stehen können;
b) sich Strahlung in bzw. entlang dem Wellenleiter ausbreitet, um die Kombination aus Analyt und immobilisiertem Reaktionsmittel zu bestrahlen; und
c) die Signalstrahlung, die aus der bestrahlten Wechselwirkung von Analyt und Reaktionsmittel resultiert, durch Beobachtung des Fluids erfaßt wird.

**Fig. 1**

**Fig. 2**

**Fig. 3**

**Fig. 4**